# EUROPEAN PATENT APPLICATION

(11) **EP 4 775 148 A1**
(43) Date of publication of application: **15.07.2026**
(21) Application number: 25150994.9
(22) Date of filing: 09.01.2025
(51) Int. Cl.: A61B 6/46, A61B 6/00

(54) **RADIOGRAPHY SYSTEM**

(71) Applicant: Koninklijke Philips N.V., 5656 AE Eindhoven (NL)
(72) Inventor: WEISS, Steffen, Eindhoven (NL)
(74) Representative: Philips Intellectual Property & Standards

(57) **Abstract**

The subject-matter of the present disclosure relates to a radiography system (100). The system comprises an imaging device (101) configured to expose a target area (106) to ionizing radiation and capture a radiographic image of the target area, an audio sensor (112) disposed proximate to the target area, and a controller (122) configured to receive an audio signal from the audio sensor, analyse the audio signal, and control an operating state of the imaging device based on the analysed audio signal.

## Description

### FIELD OF THE INVENTION

The subject-matter of the present disclosure relates to a radiography system. In particular, the present disclosure relates to a radiography system which allows for automatic control of imaging.

### BACKGROUND OF THE INVENTION

Many scans which rely on ionization radiation, such as a wall stand Xray, must be done in full inhalation. Current ionizing radiography systems are not equipped with any respiratory triggering mechanism, so staff have to give breathing commands to the patient via a patient intercom; e.g., breath hold at full inhalation. Staff, which are usually controlling the system from a control station, have no means to check for the correct breathing state of the patient when the exposure is done. This manual procedure requires time and attention by staff and patient, and it is prone to error for non-compliant patients leading to compromised image quality and unnecessary X-ray exposure.

It is therefore now desired to provide an alternative XR apparatus which improves upon the prior art.

### SUMMARY OF THE INVENTION

According to a first aspect of the present invention, there is provided a radiography system. The system comprises an imaging device configured to expose a target area to ionizing radiation and capture a radiographic image of the target area, an audio sensor disposed proximate to the target area, and a controller configured to receive an audio signal from the audio sensor, analyse the audio signal, and control an operating state of the imaging device based on the analysed audio signal.

In an example, the controller is configured to activate an interlock based on the analysis of the audio signal.

In an example, the interlock comprises one of preventing activation of the imaging device and interrupting a current activation of the imaging device.

In an example, the controller is configured to maintain the interlock for a predetermined time after determining that a condition which activated the interlock is no longer satisfied.

In an example, analysing the audio signal comprises determining a probable cause of the audio signal. In a related example, the predetermined time for maintaining the interlock is based on the probable cause of the audio signal.

In an example, the controller is configured to receive image data from the imaging device, and to add a flag to image data captured within a predetermined time frame preceding the activation of the interlock. In a related example, a length of the predetermined time frame is based on the probable cause of the audio signal.

In an example, analysing the audio signal comprises determining if the audio signal is above a predetermined threshold volume level.

I n an example, analysing the audio signal comprises inputting the audio signal to a machine learning model trained to detect at least one of talking, coughing, or sneezing, based on the audio signal.

In an example, the system further comprises a control station for controlling an operation of the imaging device.

In an example, the system further comprises a radiation shield separating the control station and the target area.

In an example, the system further comprising means for two-way communication between the target area and the control station. In an example, the aforementioned audio sensor forms a part of the means for the two-way communication.

In another aspect of the present invention, there is provided an interlock for a radiographic imaging apparatus. The interlock comprises an audio sensor disposed proximate to a target area to be exposed to radiation by the radiographic imaging apparatus, and a controller configured to receive an audio signal from the audio sensor, analyse the audio signal, and control an operating state of the radiographic imaging apparatus based on the analysed audio signal.

These and other aspects of the present invention will be apparent from and elucidated with reference to the embodiment(s) described hereinafter.

### BRIEF DESCRIPTION OF DRAWINGS

The embodiments of the present inventions may be best understood with reference to the accompanying figures, in which:
Fig. 1 shows a schematic of an example system;
Fig. 2 shows an example of audio analysis for an interlock;
Fig. 3 shows another example of audio analysis for an interlock;
Fig. 4 shows another example of audio analysis for an interlock;
Fig. 5 shows another example of audio analysis for an interlock.

### DETAILED DESCRIPTION OF EMBODIMENTS

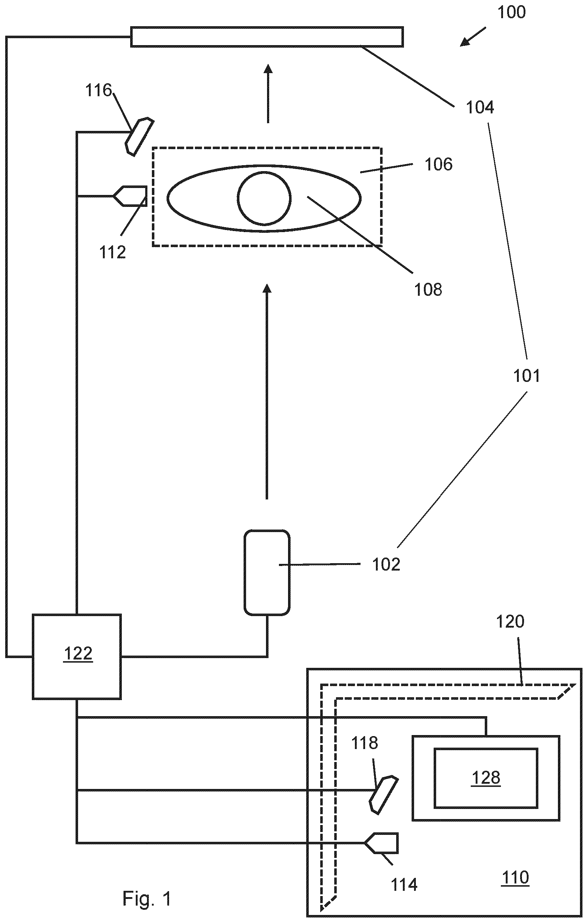

With reference to Fig. 1, there is shown an example radiography system 100. The radiography system 100 is based around the emission of ionising radiation and the subsequent imaging of that radiation. For example, the radiography system 100 may be embodied as an Xray imaging system, a computed tomography (CT) scan system, a positron emission tomography (PET) scan, and other variants of imaging systems using ionizing radiation.

Suitably, the apparatus 100 comprises a radiographic imaging device 101; for example, comprising an ionising radiation generator 102 and a radiographic image sensor 104. For example, the radiation generator 102 may be configured to generate X-rays and the image sensor 104 correspondingly configured to capture an X-ray image. If the generator 102 is configured to generate a different form of ionising radiation, the image sensor 104 is configured accordingly. As will be familiar to those in the art, the arrangement of the radiographic imaging means 102, 104 defines a target area 106 in which a target 108, such as a patient, or part thereof, may be positioned in order to capture a radiographic image based on the penetration of radiation through the target 108 in the target area 106. Put another way, it may be considered that the image sensor 104 is configured to capture a radiographic image of the target area 106.

The system 100 may comprise a control station 110, from which control parameters and functions of the radiographic imaging device 101 may be set, and which may comprise a display 128 for viewing images captured by the radiographic imaging device 101. The control station is disposed separated from the target area 106 of the radiographic imaging device 101 so as to limit the potential for exposure of an operator of the system 100 to radiation. In some examples the control station 110 is shielded from the imaging device 101 (more specifically, the ionising radiation generator 102 and target area 106 therefor) by a radiation shield 120.

Due to the separation between operator and patient 108, the system 100 may comprise means for two-way communication between control station 110 and target area 106. Using such a system the operator may instruct the patient to perform a breath hold (and allow for other communication of course).

Suitably, the two-way communication system comprises a first audio sensor 112 disposed proximate to the target area 106, and a second audio sensor 114 disposed at the control station 110. Relatedly, the two-way communication system may comprise a first audio output 116 again disposed proximate to the target area 106, and a second audio output 118 disposed at the control station 110.

The system 100 comprises a controller 122 (e.g., a processor) configured to control an operating state of the imaging device. One example operating state is an image capturing state, whereby the device is currently active in capturing an image; for example, controlling the radiation generator 102 to emit radiation and the image sensor 104 to capture an image of the received radiation after travelling through the target area 106. Another example operating state is an idle state, in which the imaging device is waiting ready to capture an image when suitably triggered to do so (e.g., by a user). A yet further example operating state is an interlocked state, which is similar to the idle state except that the radiographic imaging device is prevented from capturing an image even if triggered to do so. More specifically, an interlock in the current context may comprise one or both of preventing a new activation of the imaging device (e.g., preventing emission from the generator 102 and/or image capture by the sensor 104) and also interrupting a current activation of the imaging device (e.g., stopping further emission of radiation and further image capture). It will be appreciated however that this list of operating states is not exhaustive and the disclosure is not limited thereto.

The controller 122 is also configured to receive an audio signal from the first audio sensor 112. However, the system 100 is not limited thereto, and the controller 122 may also be configured to receive an audio signal from another, different, audio sensor positioned proximate to the target area 106 (that is not part of the two-way communication system).

Based on an analysis of the received audio signal, the controller 122 is configured to control an operating state of the imaging device 101. In particular, the controller 122 may be configured to change an operating state of the imaging device 101. For example, the controller 122 may change the imaging device 101 from the idle state to one of the active state or the interlock state, or from the active state to the interlock state, or from the interlock state to the idle state or active state, however this list is non-exhaustive.

The examples discussed herein are primarily concerned with providing an interlock for the radiographic system 100, and therefore changing operating state to/from the interlock state based on the audio signal analysis. This is because sounds coming from the patient 108 (and more generally the target area 106) can be an indicator of motion in the target area 106, which would therefore result in low quality images (and cause the patient 108 to be exposed to radiation for no useful reason).

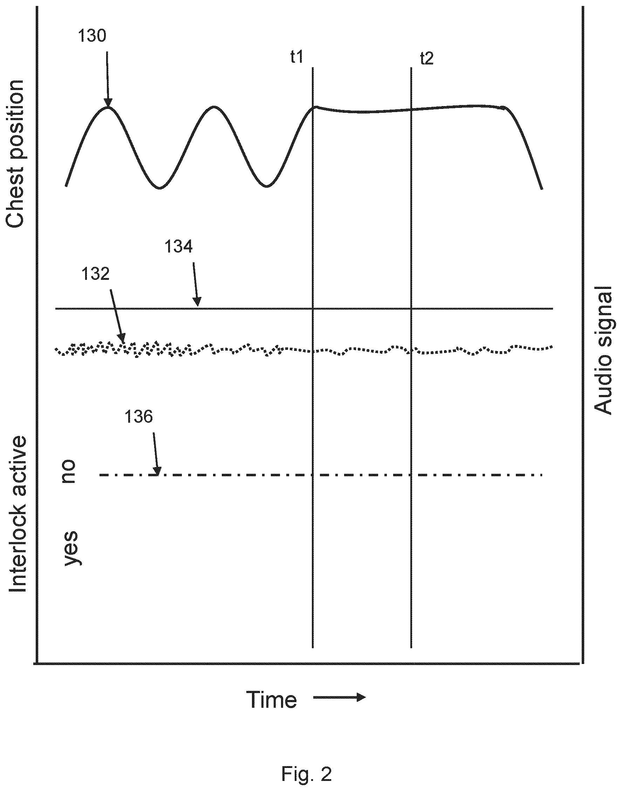

With reference to Figs. 2 to 5, there are shown examples of controlling the operating state of the imaging device 101 (in particular, activating or deactivating an interlock for the imaging device 101) based on analysis of an audio signal.

In the following examples, the audio signal analysis may comprise determining a probable cause of a sound causing a particular audio signal. For example, the analysis may comprise determining whether the source of an audio signal is one of talking, coughing, or sneezing.

The audio analysis may also comprise determining whether the audio signal is above or below a predetermined volume threshold, with the interlock being engaged when the detected sound is too high. Other criteria for engaging the interlock may also be employed.

In some examples, the audio analysis may be performed by a suitably trained machine learning model. That is, a machine learning model trained to classify a sound signal as one of talking, coughing, or sneezing. The machine learning model may also determine whether the signal is above or below a volume threshold, or may be trained to identify other features within the audio signal. The interlock may then be engaged or released based on the classification and threshold analysis given by the machine learning model, or in some examples the machine learning model may be trained to also provide an interlock on/off signal (i.e., the signal analysis remains as hidden layers within the network, and the model output is merely on/off for the interlock).

Training machine learning models for audio analysis is at this stage reasonably well known in related fields and therefore a detailed description of the training and inference of such a model is omitted.

Other techniques for audio signal analysis may also be employed, as will be appreciated by those in the art.

Fig. 2 shows an example of a baseline (idealised) image capturing process using the system 100. Here a line 130 shows a chest position of the patient 108 and initially indicates normal respiratory motion. At time t1 the patient 108 is instructed to breath hold such that their chest position remains relatively still. During this period an audio signal 132 indicates that there is little noise (for example, being below a volume threshold 134) such that the imaging device 101 remains generally in an idle state ready - i.e., there is no interlock, as shown by line 136. At time t2 the operator controls the imaging device 101 to capture an radiographic image.

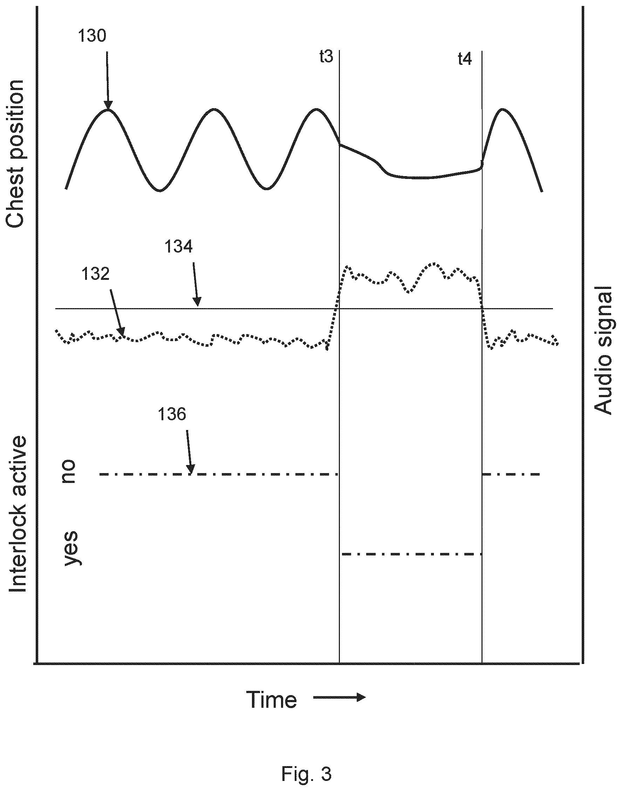

Fig. 3 shows one example of interlock activation for the system 100. Here the patient 108 is talking between a time t3 and t4. Suitably, during this time period the interlock on the imaging device is engaged (i.e., data acquisition is not allowed), as shown by line 136. Although it should be appreciated that the example may be applied to other sound sources giving rise to an audio signal which engages the interlock.

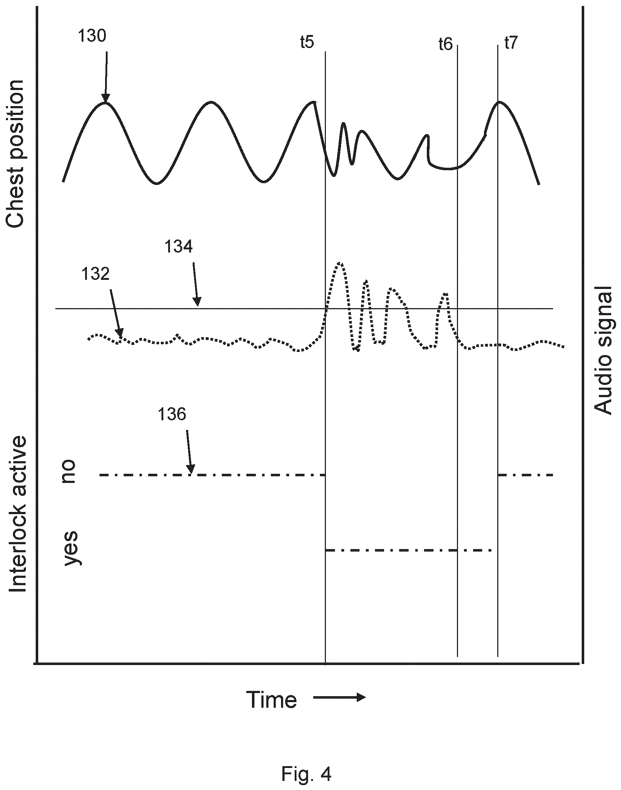

Fig. 4 shows another example for engaging the interlock for the system 100. Here the patient 108 undergoes a coughing fit between a time t5 and t6, with corresponding peaks in the audio signal 132. In this example, the controller 122 is configured to maintain the interlock on the imaging device 101 for a predetermined time after the condition which activated the interlock is no longer satisfied. For example, assuming a volume threshold is the condition for the interlock, the interlock is maintained between coughs because there is an inbuilt delay on the interlock release (that is, a delay on allowing a return to an idle and thereafter active state of the imaging device 101). Relatedly, the controller 122 may also be configured to adjust the length of the predetermined time for maintaining the interlock based on the determined probable cause of the audio signal. That is, in this example, having determined that the cause of the audio signal is likely coughing, then the controller 122 maintains the interlock for a period until a time t7, after which the interlock is released and an image can be captured.

It will again be appreciated that the example is not limited thereto, and adding delays to the interlock release may be applied in the context of other identified sound sources other than just coughing. Where the predetermined time is further based on the type of sound, the delay on interlock release may varied as appropriate to account for expected resettling time of a patient after a particular cause of sound and corresponding motion.

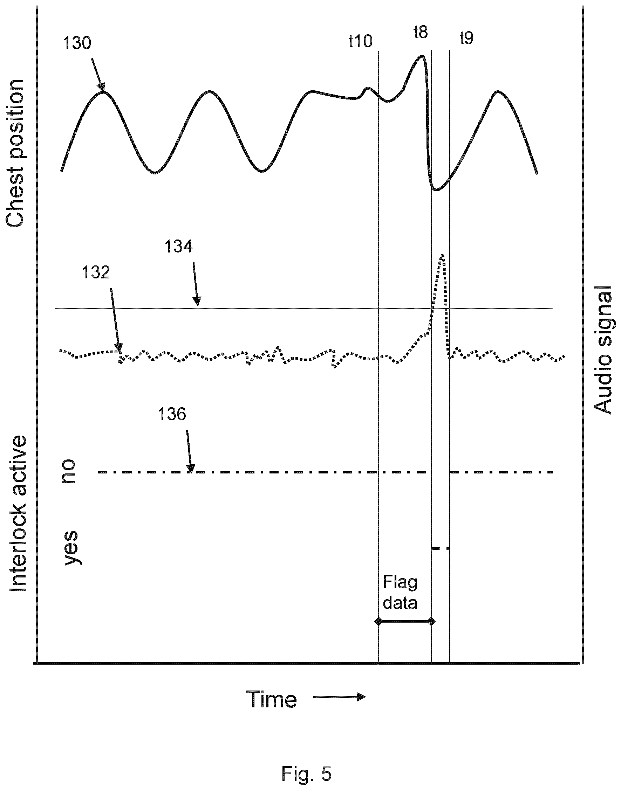

Fig. 5 shows another example for engaging the interlock for the system 100. Here the patient may sneeze between a time t8 and t9 (or cause some other sound corresponding to motion). The interlock can be released after the detection of the sneeze consistent with the examples above.

In this example however the controller 122 is configured to flag any image data that was received during a predetermined time frame preceding activation of the interlock; that is, any image data that was received from the imaging device between a time t10 and the time t8. More specifically, the controller 122 is configured to edit image data received between the time t10 and t8 to include the flag; for example, by changing one or more pixel values in the image data, or by adding metadata to the image data.

The flag can be applied to any data captured within the predetermined time, as a warning indicator to the operator to assess the data more carefully. In some examples, however, a flag may only be applied to data captured within the predetermined time frame that also corresponds to particular kinds of determined sound source. For example, in the case of talking, it is unlikely that the talking will have had an effect on previously captured data, however other causes of motion/sound like sneezing can often have motions which come before a sound is detectable. Moreover, a length of the time frame in which captured data is flagged may be varied depending on the determined probably cause of the sound.

In CT imaging particularly, mostly image data acquired over an extended period of time is included into the reconstruction of an image. The flagged data may be used differently in reconstruction in order to minimize the amount of image artefacts. Various options for using the flagged data differently are known in the art. In one example, flagged data may be excluded from image reconstruction, and the system may reacquire these missing data. In another example, the data may be used but a different algorithm may be used for reconstruction that compensates for motion. Such an algorithm may exploit the knowledge of the type of motion and the duration of the flagging period.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A radiography system (100), comprising:
an imaging device (101) configured to expose a target area (106) to ionizing radiation and capture a radiographic image of the target area,
an audio sensor (112) disposed proximate to the target area, and
a controller (122) configured to receive an audio signal (132) from the audio sensor, analyse the audio signal, and control an operating state of the imaging device based on the analysed audio signal.

2. The system of claim 1, wherein the controller is configured to activate an interlock (136) based on the analysis of the audio signal.

3. The system of claim 2, wherein the interlock comprises one of preventing activation of the imaging device and interrupting a current activation of the imaging device.

4. The system of claim 3, wherein the controller is configured to maintain the interlock for a predetermined time after determining that a condition which activated the interlock is no longer satisfied.

5. The system of any preceding claim, wherein analysing the audio signal comprises determining a probable cause of the audio signal.

6. The system of claim 5 when also dependent on claim 4, wherein the predetermined time for maintaining the interlock is based on the probable cause of the audio signal.

7. The system of any of claims 2 to 6, wherein the controller is configured to receive image data from the imaging device, and to add a flag to image data captured within a predetermined time frame preceding the activation of the interlock.

8. The system of claim 7 when also dependent on claim 4, wherein a length of the predetermined time frame is based on the probable cause of the audio signal.

9. The system of any preceding claim, wherein analysing the audio signal comprises determining if the audio signal is above a predetermined threshold volume level.

10. The system of any of claims 1 to 9, wherein analysing the audio signal comprises inputting the audio signal to a machine learning model trained to detect at least one of talking, coughing, or sneezing, based on the audio signal.

11. The system of any preceding claim, further comprising a control station for controlling an operation of the imaging device.

12. The system of claim 11, further comprising a radiation shield separating the control station and the target area.

13. The system of claim 11 or 12, further comprising means for two-way communication between the target area and the control station.

14. The system of claim 13, wherein the audio sensor forms a part of the means for the two-way communication.
